# EUROPEAN PATENT SPECIFICATION

(11) **EP 3 420 822 B1**
(45) Date of publication and mention of the grant of the patent: **31.03.2021**
(21) Application number: 18177738.4
(22) Date of filing: 14.06.2018
(51) Int. Cl.: A61P 17/06, A61P 19/02, A23L 33/115, A23L 33/12, A23D 9/02, A23D 9/04, A23D 7/02, A23D 7/04, A61K 45/06, A61K 35/60, A61K 36/63, A61P 1/16, A61P 9/10, A61K 31/202

(54) **COMBINATION OF OILS**
KOMBINATION VON ÖLEN
COMBINAISON D'HUILES

(30) Priority: 23.06.2017 NO 20171033
(43) Date of publication of application: 02.01.2019
(73) Proprietor: Arctic Omega-3 Norway AS, 9256 Tromsø (NO)
(72) Inventor: Østerud, Bjarne, 9013 TROMSØ (NO)
(74) Representative: Zacco Norway AS

(56) References cited:
- EP-A2- 0 304 115
- WO-A1-2004/002234
- WO-A1-2005/046669
- WO-A1-2006/117164
- WO-A1-2017/009874
- WO-A2-2010/055419
- CA-C- 1 336 867
- FR-A- 573 719
- GB-A- 207 545
- US-A1- 2009 264 520
- GUPTA V., MAH X.-J., GARCIA M.C., ANTONYPILLAI C., VAN DER PORTEN D.: "Oliy fish, coffee and walnuts: Dietary tretament for nonalcoholic fatty liver disease", WORLD JOURNAL OF GASTROENTEROLOGY, vol. 21, 7 October 2015 (2015-10-07), pages 10621-10635, XP002786450,
- F. VISIOLI, M. FRANCO, E. TOLEDO, J. LUCHSINGER, W.C. WILLETT, F.B. HU, M.A. MARTINEZ-GONZALEZ: "Olive oil and prevention of chronic diseases: summary of an international conference", NUTRITION, METABOLISM & CARDIOVASCULAR DISEASES, vol. 28, 26 April 2018 (2018-04-26), pages 649-656, XP002786457,
- YU TIAN & AL.: "Perilla oil has similar protective effects of fish oil on high-fat diet-induced Nonalcoholic fatty liver disease and gut dysbiosis", BIOMED RESEARCH INTERNATIONAL, 9462571, 11 February 2016 (2016-02-11), Retrieved from the Internet: URL:https://www.ncbi.nlm.nih.gov/pubmed/27 051672 [retrieved on 2019-11-08]

## Description

### FIELD OF INVENTION

The present invention relates to a combination of oils as a supplement to, or component of, a regular diet to counteract the development of coronary heart disease (CHD), thrombosis and other inflammatory diseases such as psoriasis, rheumatoid arthritis, osteoarthritis, fibromyalgia etc. The combination according to the invention comprises herring oil and cold-pressed virgin olive oil, in mixtures with omega-3 fatty acids enriched in DHA and DPA from fish oil.

### BACKGROUND OF THE INVENTION

Atherosclerotic lesions are formed when three cellular components of the circulation, monocytes, platelets and T-lymphocytes, react with LDL-cholesterol and two cell types in the artery wall, endothelial cells (EC) and the smooth muscle cells (SMC).

The precursor of atherogenesis is the recruitment of monocytes and lymphocytes from the peripheral blood to the intima of the vessel wall, an event that appears to depend upon the local presence of large amounts of LDL. As LDL accumulates, bound lipid and protein are oxidized and glycosylated. Cells in the vessel wall seem to interpret this change as a danger signal and call for reinforcements from the body's defence system. These processes appear to promote an up-regulation of adhesion molecules on the endothelial cells, particularly vascular cell adhesion molecule-1 (VCAM-1) and intracellular adhesion molecule-1 (ICAM-1). Thus, monocyte and lymphocyte recruitment is initiated. This leads to increased transmigration of monocytes, up-regulated exposure of adhesion molecules on the endothelium, and the production and release of chemoattractants. These are essential events for the transfer of monocytes to the intima, and the concurrent differentiation of monocytes into macrophages. Available modified LDL is also a prerequisite for the further development of macrophages into foam cells (fatty macrophages), which is the main cause of the formation of fatty streaks under the endothelium of the vessel wall. Modified forms of LDL (oxidized, glycosylated etc.) are of particular interest since the modification of LDL is associated with inflammatory reactions triggered by processes that are initiated due to the adhesion and transmigration of monocytes and lymphocytes into the intima.

As mentioned above, it is well known that monocytes play a central role in the early phase of atherogenesis. One of the first events in the atherosclerotic process is the mobilization of monocytes into the intima. Since the recruitment of monocytes and their penetration through the endothelium are associated with the secretion of activation products such as cytokines and growth factors, it may be assumed that the functional reactivity of the circulating monocytes is very important. It is suggested that chronic infectious diseases may affect the functional reactivity by activating the monocytes and making them more liable to produce and release harmful products such as cytokines and chemokines in response to stress.

To date, little is known as to exactly how the functional properties of circulating monocytes relate to atherogenesis. However, it is well established that hyperactive monocytes play a crucial role in the pathophysiology of rheumatism, psoriasis and other inflammatory diseases. We also know that atherogenesis is a pro-inflammatory disease. It may therefore be assumed that the pro-inflammatory function of circulating monocytes may be associated with increased risk of coronary heart disease (CHD), and that high cholesterol levels may augment production of pro-inflammatory products such as oxygen radicals, cytokines etc.

For many years, the inventor has observed that the reactivity of monocytes, as monitored by the production of tissue factor (TF) and cytokines such as TNFa and IL-6 in lipopolysaccharide (LPS) stimulated blood, varies between individuals from low activity to very high activity (high responders). This property of monocytes seems to be hereditary (Østerud et al, "Blood Coagulation and Fibrinolysis" 2002; 13:399-405). The inventor has, *inter alia,* investigated *in vitro* howLPS-induced reactivity in monocytes in whole blood relates to the lipid profile in the serum of healthy individuals with a history of myocardial infarction (Ml) or cancer in their close family. Of a total of 54 individuals in the myocardial infarction (Ml) families, 20 had moderately high cholesterol (7.1 -10.2 mmol/1), whilst 34 had normal cholesterol. Of the individuals with normal cholesterol, 19 had hyperactive monocytes (high responders), whilst 15 had normally responding monocytes. LPS-induced TF, TNFa and IL-6 were on average 3-4 times higher in the group with normal cholesterol compared with the group with moderately high cholesterol. Thus, no positive correlation between hyperactive monocytes and cholesterol level was found. All 42 individuals in the families with a history of cancer had normal cholesterol, and LPS-induced thromboplastin (TF), TNFa and IL-6 were not significantly different from the values of the group with moderately high cholesterol among the myocardial infarction (Ml) families. This supports the conclusion that moderately high cholesterol is not associated with increased monocyte activation in whole blood, whilst hyperactive peripheral blood monocytes are a significant risk factor for the development of coronary heart disease.

It is probably at least as important to reduce the reactivity of monocytes, and thus the production of pro-inflammatory products such as cytokines, oxidative metabolites and growth factors, as it is to reduce the cholesterol level. New studies also show that the anti-inflammatory effect of statins may be more important than their cholesterol reducing effect (Balk et al. "Effects of statins on nonlipid serum markers associated with cardiovascular disease: a systematic review" Ann Intern Med. 2003; 139:670-82. Review).

The worldwide epidemic increase in obesity associated with energy-rich diets with sugar and saturated fats has led to a strong increase in so-called "non-alcoholic fatty liver disease" (NAFLD). NAFLD is considered to be the most serious form of chronic liver disease. Non-alcoholic steatohepatitis (NASH) is the most serious form of NAFLD, characterized by steatosis associated with inflammation, which can develop into liver fibrosis.

It is today the second most common cause of liver transplantation in the United States of America and is expected to be the leading cause by 2020. No single treatment has been approved for treatment of NASH.

Omega-3 fatty acids are known to reduce the risk of arrhythmia which can lead to sudden death. Omega-3 fatty acids are also known to reduce the risk of thrombosis which can lead to heart attack and stroke. They reduce the growth rate of atherosclerotic plaque, and thus have anti-inflammatory properties as lesion formation in the atherogenic process is mediated by pro-inflammatory reactions. Furthermore, omega-3 fatty acids improve endothelial function, reduce the level of triglycerides in the blood and lower the blood pressure slightly (for a brief overview, reference is made to PM Kris-Etherton, WS Harris, LJ Appell "Arterioscler Thromb Vase Biol." 2003;23:151-2).

In view of the properties of omega-3 fatty acids, it would be expected that a supplement of omega-3 fatty acids ought to be sufficient to prevent cardiovascular disease. However, clinical studies carried out in Norway have shown negative effects of omega-3 fatty acids (I Seljefot, 0 Johansen, H Arnesen, JB Eggesbo, AB Westvil, P Kierulf, "Thromb Haemost." 1999; 81:566-70; O Johansen, I Seljefot, AT Hostmark, H Arnesen "Arterioscler Thromb Vase Biol." 1999; 19; 1681-6). Patients with cardiovascular disease who were given a supplement of omega-3 fatty acids for six months experienced a doubling of both angina and occlusions compared with controls. An increase in cytokine production was also observed, which is indicative of an increase in pro-inflammatory peroxidation of polyunsaturated fatty acids *in vivo* (for an overview, reference is made to H. Arnesen, "Lipids" 2001; 36 Suppl: S103-6).

The findings referred to above are in accordance with the inventor's own results with respect to diets including a supplement of omega-3 fatty acids. Thus, the anti-inflammatory effect of supplementing the diet of healthy individuals with an omega-3 fatty acid concentrate was not significant compared with a corresponding amount of omega-3 fatty acids in the form of cod-liver oil (CLO).

Although, many attempts have been made to find ways to prevent and treat the disorders and diseases discussed above, and a number of products are offered in this respect, there is still a need to provide improved and more effective products.

WO 2006/118463 discloses a combination of seal oil and cold-pressed virgin olive oil showing potential to increase the beneficial HDL cholesterol, reducing the marker and risk factor for CHD, as well as reducing pro-inflammatory products.

WO 2010/149815 discloses functional oil based on olive oil (87-96 %) and one or more ingredients selected from: avocado oil, argan oil, safflower oil, wheatgerm oil, krill oil, linseed oil, walnut oil, pumpkin seed oil, propolis oil, sesame oil and soya isoflavone; coenzyme Q-10; vitamin E; vitamin D; lycopene; vitamin K; vitamin A; EPA, DHA and mixtures thereof. Use of said functional oil as a medicinal product for the treatment and/or prophylaxis of *inter alia* coronary diseases, cardiovascular diseases (e.g. hypertension and thrombosis), inflammatory processes and atherosclerosis are described.

Yang et al. ("Dietary marine-derived long-chain monounsaturated fatty acids and cardiovascular disease risk: a mini review", Lipids in Health and Disease (2016) 15:201) reports that regular fish/fish oil consumption is widely recommended for protection of cardiovascular diseases (CVD). Fish and other marine life are rich sources of the cardioprotective long-chain n-3 polyunsaturated fatty acids (n-3 PUFA) eicosapentaenoic acid (C20:5 n-3, EPA) and docosahexaenoic acid (C22:6 n-3; DHA). The lipid content and fatty acid profile of fish, however, vary greatly among different fish species. In addition to n-3 PUFA, certain fish, such as saury, pollock and herring, also contain high levels of long-chain monounsaturated fatty acids (LCMUFA), with aliphatic tails longer than 18 C atoms (i.e. C20:1 and C22:1 isomers). A limited number of *in vivo* animal studies have provided valuable evidence supporting the potential for LCMUFA rich diet in the prevention of life-style related diseases, such as type 2 diabetes, metabolic syndrome and atherosclerosis. A few human studies have also suggested a possible link between LCMUFA-rich diet and CVD risk protection.

In addition, B. Ruyter and A. Nilsson, researchers at Nofima have found that feeding salmon with herring oil rich in the 22: 1(n-11) fatty acid, cetoleic acid, reduced fatty liver in salmons.

EP 0 304 115 A2 discloses an edible composition comprising blend unhydrogenated marine oil and vegetable oil in a ratio of not more than 1:3.

WO 2006/117164 A1 discloses a food supplement or functional food comprising:
(a) a nut oil in an amount of 0.5 to 20% vol., (b) a source containing ω-3 fatty acids (e.g. fish oil) in an amount of 10 to 50% vol., wherein the total amount of ω-3 fatty acids is at least 20% vol., and (c) a flavour carrier in an amount of 10 to 89.5% vol. (e.g. olive oil).

Now, the inventor has surprisingly found that by combining cold-pressed virgin olive oil and herring oil, and one or more omega-3 fatty acids selected form DHA (docosahexaenoic acid (22:6 n-3)), EPA (eicosapentaenoic acid (20:5 n-3)) and DPA (docosapentaenoic acid (22:5 n-3)), a product exhibiting enhanced effect with regard to prevention and/or treatment of at least atherosclerosis, is obtained.

### SUMMARY OF THE INVENTION

It is a main object of the present invention to provide a food/dietary supplement to counteract the development of CHD, thrombosis and other inflammatory diseases such as psoriasis, rheumatoid arthritis, osteoarthritis, fibromyalgia etc.

Another object of the present invention is to provide a combination for use in treatment or prophylaxis of atherosclerosis, CHD, thrombosis and other inflammatory diseases such as psoriasis, rheumatoid arthritis, osteoarthritis, fibromyalgia etc.

These and other objects are obtained by the combination as defined in the accompanying claims.

### DETAILED DESCRIPTION OF THE INVENTION

The present invention provides a combination of oils comprising 33% of herring oil, 33% of cold-pressed virgin olive oil and 33% of omega-3 fatty acids enriched in DHA and DPA from fish oil.

In a preferred embodiment of the invention, the combination comprises omega-3 fatty acids selected from EPA, DHA, and DPA to give a final total concentration of the omega-3 fatty acids in the combination of more than 1 gram per day.

Furthermore, the present invention relates to the use of the said combination as a food supplement, dietary supplement, an edible oil, or a component of an oil-in-water or water-in-oil emulsion in foodstuffs.

In another aspect the present invention provides the said combination for use in treatment or prophylaxis of diseases or disorders selected from the group consisting of atherosclerosis, coronary heart disease, thromboses, psoriasis, rheumatoid arthritis, osteoarthritis, fibromyalgia and other pro-inflammatory diseases.

The combination according to the invention combines the effect of omega-3 fatty acids, monounsaturated fatty acids and a synergistic component which gives an antioxidation effect both *in vivo* and *in vitro.* This combination has advantageous properties in the form of better functional properties and longer storage life. The inventor has shown that a particularly advantageous effect is obtained using a product that comprises oil from herring and cold-pressed virgin olive oil, both of these components being manufactured in a known manner. The effect obtained is to be more pronounced than would be expected if each component were used alone.

Thus, the invention relates to a combination of oils as a supplement to a regular diet, comprising a combination of herring oil and cold-pressed virgin olive oil with added additionally omega-3 fatty acids, DHA, EPA and DPA.

What distinguishes herring oil from most other marine oils is that, in addition to comprising the omega-3 fatty acids EPA and DHA, it is rich in the long-chain monounsaturated fatty acids 20:1 and 22:1.

The cold pressed olive oil used in the combination of the invention, is characterized in that it has a great potential to prevent oxidation of polyunsaturated fatty acids measured in Oxidograph at 70 °C when the olive oil is combined with marine oils rich in omega-3 fatty acids. The qualities of olive oil are mainly due to its high content of monounsaturated fatty acids, especially oleic acid (18:1 n-7, n-9), vitamins A,E, D and K, and polyphenols with antioxidant activity (hydroxytyosol, tyrosol) secoirdoides (oleuropein), lignans (acetoxipinoresinol, pinoresinol, flavones, pigments and beta-carotene. That is, it is very rich in highly potent fat-soluble antioxidants. The olive oil in the combination of the invention is also distinguished by the high level of the antioxidant oleocanthal which gives the combination of the invention a very characteristic bitter taste and this component in olive oil has been shown to have a number of interesting effects in itself (Parkinson and Keast, Int. J. Mol. Sci. 2014; 15, 12323 -12334, Review).

The invention is explained in more detail in the examples below.

### EXAMPLES

### Example 1 (reference example)

A mouse study was conducted at TNO, Leiden, The Netherlands on the effect of adding the combination of the invention based on the combination of refined herring oil and cold-pressed virgin olive oil to the diet and comparing the effects with corn oil.

The ratio of herring oil to cold-pressed virgin olive oil in the combination of the invention used in the study was 50:50. A total amount of 1% of the oils was added to the diet.

In the control, 1 % of corn oil was added to the diet.

The study was done by comparing the effect of the nutrient oil on reducing the development of lesions (atherosclerosis) in apoE - / - mice (Apo3Leiden mice) fed with a fat diet with 0.25% cholesterol for 20 weeks as well as on the cellular and biochemical processes involved in the development of atherosclerosis with a corn oil as a control. At first, the mice were raised to the age of 12 weeks where the mice were put on a 3 weeks high cholesterol diet with 15% saturated fat and 1% (w / w) cholesterol. There were 20 mice in each group.

The results of the nutrient effect on lesions (atherosclerosis) showed that the combination of the invention reduced the formation of lesions by 49% (p <0.001).

### Example 2 (reference example)

TNO, Leiden, The Netherlands, has developed, along with human liver pathologists, two validated diet-induced NASH models that also develop liver fibrosis over time and a general measurement system for female mice (Liang et al., Lab Invest 2014, 94: 491-502; Liang et al. All PLoS ONE 2014 9 (12): e115922). The models have mapped the time in the development of the disease and mimic all the features of human pathology. NASH begins to develop after 12-16 months and liver fibrosis after 20-24 months.

Since inflammation is an essential factor in the development of this disease, it was of great interest to test both scientifically and, not least, the health aspect of the NASH models of the combination of the present invention with a very potent anti-inflammatory effect, to see whether the invention oil could hinder the development of NASH and liver fibrosis. As a control, corn oil was used. The same concentrations of the oils as indicated in Example 1 were used in this study.

The mice used for the studies of atherosclerosis in Example 1 were also analysed for liver injury, NASH and fibrosis measurements by histopathology after 20 weeks of feeding.

The results of the study showed:
25% reduction in so-called macrovascular steatosis (p <0.003)
23.3% reduction in microvascular steatosis
33.9% reduction in hypertrophy
Liver lipids decreased by 72.9% (p = 0.026)

Despite the fact that the NASH model was not optimal in this case (should be used higher amounts of fat that makes it more sensitive), the study showed that the combination of the invention has a very beneficial effect to reduce NASH. A reduction on NASH is very important in limiting the damaging effect of overweight that is constantly increasing throughout the world.

The conclusion is that the combination of the invention has the potential to prevent atherosclerosis and to reduce the harmful effects of fatty liver caused by increased fat intake.

### Example 3

In this example, the APOE*3Leiden.CETP mice was used instead of the APOE*3Leiden as the APOE*3Leiden.CETP mice are more sensitive to cholesterol and more similar to humans. This study was also conducted at TNO, Leiden, The Netherlands.

In this study, mean atherosclerotic lesion size was 370*10³ µm² per cross section, whereas in Example 1, the lesion size was 85*10³ µm², which means that the atherosclerotic process was more advanced in Example 3.

The ratio of herring oil to cold-pressed virgin olive oil in the combination of the invention used in the study was 50:50. A total amount of 1% of the oils was added to the diet. The effect of this invention blend was compared to the combination of seal oil and cold-pressed olive oil in WO 2006/118463. In addition, the effect of adding an omega-3 concentrate, particularly rich in DHA and DPA, was tested. This combination oil blend contained 1:1:1 of herring oil, cold-pressed olive oil and the omega-3 concentrate.

In the control, 1 % of corn oil was added to the diet.

The study was done by comparing the effect of the nutrient oils on reducing the development of lesions (atherosclerosis) using female APOE*3Leiden.CETP mice put on a high fat cholesterol diet with 15% saturated fat and 1% (w/w) cholesterol for 16 weeks. There were 15 mice in each group.

The results of the nutrient effect on lesions (atherosclerosis) showed that the combination of herring oil and cold-pressed olive oil reduced lesions (atherosclerosis) by an average 42% (p< 0.001) compared to 31% (p< 0.05) for the combination of seal oil and cold-pressed olive oil. The herring oil combined with corn oil had a 32% (0.04) reduction.

The most superior effect of the combinations of the invention was obtained by the combination of herring oil, cold-pressed olive oil and the omega-3 concentrate as the reduction in atherosclerotic lesions was 56.7% (p<0.001) compared to the control.

The effect of the combination of oils according to the present invention on atherosclerotic lesion formation correlated well with the reduction in total cholesterol levels. The herring oil/cold-pressed olive oil had 18.7% reduction compared to 14.2% for the combination of seal oil and cold-pressed olive oil and 33.9% reduction was found for the combination of herring oil, cold-pressed olive oil and the omega-3 concentrate.

## Claims

1. A combination of oils, **characterized in that** it comprises 33% of herring oil, 33% of cold-pressed virgin olive oil and 33% of omega-3 fatty acids enriched in DHA and DPA from fish oil.

2. The use of the combination according to claim 1, as a food supplement, dietary supplement, an edible oil, or a component of an oil-in-water or water-in-oil emulsion in foodstuffs.

3. The combination according to claim 1, for use in treatment or prophylaxis of diseases or disorders selected from the group consisting of atherosclerosis, coronary heart disease, thromboses, psoriasis, rheumatoid arthritis, osteoarthritis, fibromyalgia and other pro-inflammatory diseases.

## Patentansprüche

1. Kombination von Ölen, **dadurch gekennzeichnet, dass** sie 33% Heringöl, 33% kaltgepresstes Virgin Olivenöl und 33% Omega-3-Fettsäuren, angereichert mit DHA und DPA aus Fischöl umfasst.

2. Verwendung der Kombination nach Anspruch 1 als Nahrungsmittelergänzung, Nahrungsergänzungsmittel, Speiseöl oder Bestandteil einer Öl-in-Wasser-oder Wasser-in-ÖI-Emulsion in Lebensmitteln.

3. Kombination nach Anspruch 1 zur Verwendung bei der Behandlung oder Prophylaxe von Krankheiten oder Erkrankungen ausgewählt aus der Gruppe bestehend aus Atherosklerose, koronarer Herzerkrankung, Thrombosen, Psoriasis, rheumatoider Arthritis, Osteoarthritis, Fibromyalgie und anderen proinflammatorischen Erkrankungen.

## Revendications

1. Combinaison d'huiles, **caractérisée en ce qu'**elle comprend 33% d'huile de hareng, 33% d'huile d'olive pressée à froid et 33% d'acides gras oméga-3 enrichis en DHA et DPA provenant d'huile de poisson.

2. Utilisation de la combinaison selon la revendication 1, en tant que complément alimentaire, complément de diète, huile comestible ou composant d'une émulsion huile dans eau ou eau dans huile dans les denrées alimentaires.

3. Combinaison selon la revendication 1, destinée à être utilisée dans le traitement ou la prophylaxie de maladies ou de troubles choisis dans le groupe consistant en l'athérosclérose, la maladie coronarienne, les thromboses, le psoriasis, la polyarthrite rhumatoïde, l'arthrose, la fibromyalgie, d'autres maladies pro-inflammatoires.
